# EUROPEAN PATENT SPECIFICATION

(11) **EP 3 467 770 B1**
(45) Date of publication and mention of the grant of the patent: **23.11.2022**
(21) Application number: 17194971.2
(22) Date of filing: 05.10.2017
(51) Int. Cl.: G06T 7/00, G06K 9/62, G16H 15/00, G16H 30/40, G16H 50/20, G06V 10/82

(54) **METHOD FOR ANALYSING A MEDICAL IMAGING DATA SET, SYSTEM FOR ANALYSING A MEDICAL IMAGING DATA SET, COMPUTER PROGRAM PRODUCT AND A COMPUTER-READABLE MEDIUM**
VERFAHREN ZUM ANALYSIEREN EINES MEDIZINISCHEN BILDDATENSATZES, SYSTEM ZUM ANALYSIEREN EINES MEDIZINISCHEN BILDDATENSATZES, COMPUTERPROGRAMMPRODUKT UND COMPUTERLESBARES MEDIUM
PROCÉDÉ D'ANALYSE D'UN ENSEMBLE DE DONNÉES D'IMAGERIE MÉDICALE, SYSTÈME D'ANALYSE D'UN ENSEMBLE DE DONNÉES D'IMAGERIE MÉDICALE, PRODUIT-PROGRAMME D'ORDINATEUR ET SUPPORT LISIBLE PAR ORDINATEUR

(43) Date of publication of application: 10.04.2019
(73) Proprietor: Siemens Healthcare GmbH, 91052 Erlangen (DE)
(72) Inventor: Hartung, Andre, 91052 Erlangen (DE); Ionasec, Razvan, 90402 Nuernberg (DE)

(56) References cited:
- EP-A1- 3 196 791
- DE-A1-102010 028 382
- US-A1- 2016 350 919
- US-A1- 2016 364 631

## Description

The present invention describes a method for analysing a medical imaging data set, a system for analysing a medical imaging data set, a computer program product and a computer-readable medium.

Medical imaging data sets are well known in the prior art. In general, medical imaging data sets are recorded and reconstructed by medical imaging devices, such as an X-ray scanner, computer tomography (CT) scanner, magnetic resonance tomography (MRT) scanner or ultrasound scanner, for example. However, after recording and reconstructing the medical imaging data set, a detailed analysis has to be performed by a specialist, for example by a radiologist, for identifying abnormality in the medical imaging data set. Thereby, a large proportion of these subsequent detailed analyses or exams leads to an irrelevant result due to an absence of any abnormality, i. e. a success rate of identifying an abnormality in a medical imaging data set is comparatively low. Nevertheless, those analyses or exams have to be performed, although the majority of the recorded medical imaging data sets have no indication for abnormalities. As a consequence, free capacities for analysing medical imaging data sets are reduced.

US 2016 0364631 A1 deals with methods and systems for automatically analyzing clinical images using rules and image an-alytics. One system includes a server including an electronic processor and an interface for communicating with at least one data source. In particular, US 2016 0 364 631 A1 discloses a learning engine that determines a probability for a diagnosis. Further, the learning engine may automatically identify exams that need to be sent to an external reading device.

EP 3 196 791 A1 discloses a method for assisting medical personnel in the examination of a patient, wherein the examination comprises at least one measurement. Thereby information of the patient saved in a data base is considered in the examination as well as a prevalence rate for a future examination.

US 2016 350 919 discloses systems and methods for processing electronic imaging data obtained from medical imaging procedures. Some embodiments relate to data processing mechanisms for medical imaging and diagnostic workflows involving the use of machine learning techniques such as deep learning, artificial neural networks, and related algorithms that perform machine recognition of specific features and conditions in imaging data. Based on the characteristics recognized by the automated image recognition on the image data, an electronic workflow for performing a diagnostic evaluation of the medical imaging study may be modified, updated, or prioritized. DE 10 2010 028 382 A1 deals with a method for modifying medical image data set being recorded by CT-tomography. Thereby, the pixels of the medical image data set are classified by probabilities.

It is therefore an object of the present invention to provide a method for analysing a medical imaging data set, wherein an effort for analysing medical imaging data sets is reduced and/or a success rate for identifying an abnormality in a medical imaging data set is increased.

This object is achieved by four aspects of the present invention, namely by the method according to claim 1, by the system according to claim 9, the computer program product according to claim 10 and by the computer-readable medium according to claim 11.

Contrary to the state of the art, it is provided according to the present invention that the medical imaging data set is transferred either to an output device or to a device for storing the medical imaging data set and/or for creating a report data set based on the probability value, i. e. a preselection of the medical imaging data sets is performed. As a consequence, a specialist for analysing the medical imaging data sets in detail is no longer in charge of analysing those medical imaging data sets that have a high probability of identifying no abnormality in a detailed analysis. The probability value represents a probability for successively identifying no abnormality when analysing the medical imaging data set in detail. By omitting the analysis of those medical imaging data sets having a comparatively high probability for identifying no abnormality it is further advantageously possible to increase the success rate or concentrate the analysing effort to those analyses having a certain probability of being successful in identifying an abnormality. In other words: the method according to the present invention preselects medical imaging data sets and redirects the effort for analysing the medical imaging data sets to those having a low probability for a negative finding. Subsequently, the analysed medical imaging data sets are preferably transferred to the device for storing the medical imaging data set, too. As a result of the method a rate for providing analysed medical imaging data sets to the device for storing the medical imaging data sets per time is increased, advantageously, since a comparatively high number of medical imaging data sets are transferred directly to the device for storing the medical imaging data set. Another advantage is that the specialist for analysing the medical imaging data sets can concentrate his focus on those medical imaging data sets having a reduced probability for a negative finding. Another advantage is concentrating on negative findings (instead of concentrating on positive findings), since in that way not all information being available for all potential abnormalities respectively have to be considered. Thus, comparing the medical imaging data sets to previous medical imaging data sets is simplified.

Preferably, the medical imaging data set is recorded by a medical imaging device such as a x-ray-scanner, a CT (computer tomography scanner) - scanner, a MRT (magnetic resonance tomography) - scanner or the like, and subsequently the medical imaging data set is provided, preferably to a control unit. The control unit comprises a processor being configured for at least one of the steps according to the present invention, in particular for executing at least
- providing the medical imaging data set automatically either
   - to an output device for analysing the medical imaging data set or
   - to a device for storing the medical imaging data set and/or for creating a report data set
   based on the probability value. It is thinkable that the control unit is incorporated into a medical imaging device, a workstation, such as a personal computer, and/or a server or a system of servers, such as a network or a cloud. The medical imaging data set represents a three- or four-dimensional data set. Preferably, at least one of the steps according the invention is executed on a server, in particular a cloud.

The term "output device" is preferably generic for a device being configured for presenting or depicting a visualisation of the medical imaging data set. For example, the output device is a screen depicting a visualisation of the medical imaging data set or a printing device for printing a visualisation of the medical imaging data set, for example on a sheet. In other words: the output device presents the medical imaging data set in a suitable way for analysing the medical imaging data set in detail by an operator or a clinician.

The term "device for storing" preferably describes a memory device used for storing the recorded medical imaging data sets e.g. a digital storage medium such as a hard disc, SD-card, which may be part of a computer or cloud. Preferably, the medical imaging data sets being transferred directly to the device for storing by the control unit are labelled with a standard phrase for identifying them. Furthermore, it is thinkable that the output device and the device for storing the medical imaging data set are integrated into a common structure, such as a workstation, or the device for storing the medical imaging data sets is incorporated into a server or a system of servers, whereas the output device is included in a workstation or the medical imaging device.

In particular the report data set comprises a note or a text phrase being provided to the clinician in order to inform the clinician, whether the medical imaging data set has to be analysed in Detail or not. For example, the report data set is transferred by email.

Preferably, assigning the probability value to the medical imaging data set is realized by the control unit, in particular by pre-analysing the medical imaging data set. For example, the control unit compares the recorded medical imaging data set to previous recorded medical imaging data sets having either a negative finding or a positive finding. By specifying the type of abnormality the comparison can be limited to corresponding medical imaging data sets recorded in the past or to special parts of the medical imaging data set being relevant for the finding. As a consequence, a computing effort of the control unit can be reduced, since the control unit concentrates its pre-analysis or preselection to relevant medical imaging data set and/or relevant parts of the medical imaging data set.

Particularly advantageous embodiments and features of the invention are given by the dependent claims as revealed in the following description. Features of different claim categories may be combined as appropriate to give further embodiments not described herein.

It is provided that an information data set is provided and that the probability value is based on the medical imaging data set and the information data set. By considering the information data set in addition to the medical imaging data set for determining the probability value, it is advantageously possible to further increase the success rate for having a positive finding in the detailed analysis by the specialist, since even more medical imaging data sets that need no detailed analysis can be sorted out. The term "information data set" describes data sets that compile personal information of the patient such as an age, a sex of the patent, a lab result of the patient, patient record information, a first assessment and/or previous exams. In particular, the information data set is automatically extracted from a data base such as from a RIS (RadiologieInformationsSystem), a PACS (Picture Archiving and Communication System), a EMR (ErfahrungsMedizinischen Register), a HIS (Hospital Information System), a LIS (Labor-Informationssystem) or other.

Preferably, the information data set is extracted from several data bases or constructed by several information extracted from different patient related data bases. It is also thinkable that the information data set is included in a label of the medical imaging data set and the control unit decodes the needed information from the labelling.

In particular, it is provided that the probability value is provided by a trained artificial network, wherein preferably the artificial network is trained by a machine learning mechanism, in particular a deep learning mechanism, and/or by using a Siamese algorithm. Preferably, it is provided that a data driven machine learning artificial intelligence method is used for determining the probability value. For example, an algorithm framework uses a deep learning (conventional neural network) approach that learns a similarity metric form a labelled dataset. The training approach employs a discriminative loss function that drives the similarity metric to be high for similar pairs and small for different pairs of medical imaging data sets. The convolutional neuronal network or any other data driven model is optimized to project images into a lower dimensional space robust to irrelevant differences (such as noise, artifacts, exposure, non-discriminant anatomical features) and discriminant for diseases classes relevant for a specific exam including a normal patient class. Preferably, a Siamese algorithm can be used for the training of the similarity metric.

Further, it is thinkable that a sampling rate is adapted for computing and aggregating the similarity of the recorded medical imaging data set to those saved in a training data base. For example, the labels of the closest matches will determine the probability value as well as a final rule-out label. As a consequence, the problem is reduced to a two class classification problem to discriminate between normal and any of the diseased classes.

Preferably, the sampling is adjusted to the patient information, in particular to at least a part of the information training data set. Thus, it is possible to increase a success rate for comparing the recorded medical imaging data set to previous medical imaging data sets. In particular, an interrogation of individuals in the database of the same age, sex, race, BMI or other parameters that may help eliminating biases is performed.

Preferably, it is provided that a result data set is provided after analysing the medical imaging data set and the result data set is used for training the artificial network. Preferably, the result data set is transferred to and stored at a data base of the artificial network. Thus, the artificial network is trained continuously by using the result data sets. In particular, the result data set comprises the medical imaging data set, the information data set and preferably the result of the detailed analysis. Alternatively and/or additionally artificial result data sets are incorporated to the training data base for periodically updating the artificial network.

In another preferred embodiment it is provided that analysing is supported by an analysing device for highlighting an abnormality. Thus, it is possible to use the highlighting of the abnormality for comparing the medical imaging data set being analysed in detail and subsequently stored in the training data base of the artificial network to the present medical imaging data set in order to provide the probability value. For example, an area of the highlighted region is stored together with the medical imaging data set. When the control unit identifies a potential relevant region in the medical imaging data set it is possible to compare the area and/or shape of the potential relevant region to the area and/or shape of the highlighted region of the medical imaging data sets stored in the training data base for providing a probability value. It is also thinkable that the highlighting represents anatomic landmarks and/or that the analysing device highlights identified abnormality during the pre-analysis of the control unit (i. e. the analysis device is incorporated into the control unit in such cases).

Furthermore, it is provided that the result data set and/or the medical imaging data set is transferred to a data base of a clinical decision support system. In general, such a clinical decision support system collects all information about the patient and automatically suggests for example a further treatment of the patient. In particular, it is provided that the medical imaging data set being analysed in detail and the medical imaging data sets being transferred without further analyses are stored in the data base of the clinical decision support system.

According to the present invention it is provided that the probability value is compared to a threshold value. Thus, the control unit determines whether the medical imaging data set has to be analysed in detail or can be transferred directly to the storage device. It is also thinkable that the threshold value is adaptable, in particular adaptable by the control unit. By adapting the threshold value it is advantageously possible to set the threshold value such that the probability of transferring a medical imaging data set including an abnormality directly to the device for storing the medical imaging data set is as low as possible.

According to the present invention it is also provided that a further probability value is provided based on the information data set, in particular before recording the medical imaging data set, and wherein the medical imaging data set is only automatically provided either
- to the output device for analysing the medical imaging data set or
- to a device for storing the medical imaging data set and/or for creating a report data set
based on the probability value, when the difference between the threshold value and the further probability value is smaller than a further threshold. As a consequence, it is possible to transfer those medical imaging data sets that should analysed anyway directly to the output device. For example, the information data set comprises an information about a previous disease and the probability of a return of this disease, i. e. the abnormality caused by the disease, is comparatively high. In such cases a detailed analysis should performed anyway. By skipping the step of determining whether the medical imaging data set should be transferred to the output device or the device for storing the medical imaging data set the control unit is released.

In another embodiment it is provided that the information data set is based on a patient related data base. Thus, further information of the patient can be taken into account for determining the probability value. Furthermore, it is thinkable that a class of specialist is suggested by the control unit based on the probability value. Thus, it is possible to direct difficult medical imaging data sets to experienced specialists.

Preferably, the information data set and/or the threshold value are entered via an input device. Preferably, the input device is a human machine interface such as a touch screen or a key board. Thus, it is possible to enter the information data set manually and subsequently transferring the information data set to the control unit for providing the probability value and/or further probability value.

In another embodiment of the present invention it is provided that the information data set and/or the threshold value are set automatically, in particular by using the trained artificial network. In particular, it is provided that the control unit and/or the trained artificial network access to the patient related data base such that the control unit and/or the artificial network can get additional information on demand. For example, the control unit and/or the trained artificial network need further information about the patient stored in the PACS for providing the probability value.

In an example, a pneumothorax is suspected by a clinician of an emergency department, in particular after a blunt trauma. The pneumothorax is one of the most common forms of thoracic abnormalities. For instance, the clinician evaluates a preliminary probability of a pneumothorax bigger than 10 %. In order to confirm the suspected pneumothorax a chest radiography and/or a high resolution CT is performed. In particular, a supine anteroposterior (AP) chest radiograph is performed for identifying the presence of the pneumothorax, wherein such a test has a likelihood of being negative between 0.25 and 0.72. By using the control unit it is provided that the medical imaging data set is used for identifying medical imaging data sets that are normal with a high sensitive, resulting in high probability value, and/or a likelihood of being negative close to 0. Thereby, a Mahalanobis distance might be used to quantify the variation in similarity of the current recorded medical imaging data set compared to the mean of the normal distribution in the data base of the artificial network. The threshold can be set depending on the standard deviation. For example, the threshold is set more than 1.5 times of the standard deviation away from a mean of probability values of the previous recorded medical imaging data sets. In such cases when the probability value of the current medical imaging data set is higher than the threshold value, the medical imaging data set is classified as "normal" and a standard report data set is generated. In such cases when probability value is smaller than the threshold the current medical imaging data set is redirected to the output device for being analysed by a specialist.

A standard report data file, for example an ASCII report data file, comprises the following string or phrase:
"findings:
   No pre-examination for correlation. Upper mediastinum is slim and located in the middle. Size of the heart within standards. Hilar structures are resolvable in typical way for vessels on both sides. No signs of congestion at the pulmonary venous. No pleural effusion. No indication for pneumatic infiltrates in regions of the pulmonary tracts being visible free from overlapping. No pulmonary nodules definable. No indication for pleural dehiscence. Parts of the axial skeleton being imaged seem to be unobtrusive.
estimation:
   No indication for cardinal decompensation or pneumatic infiltrates."

In the drawing:
- Fig. 1: shows a block diagram illustrating a system for analysing a medical imaging data set according to a preferred embodiment of the present invention.
- Fig. 2: shows a flow diagram illustrating a method for analysing a medical imaging data set according to a preferred embodiment of the present invention.

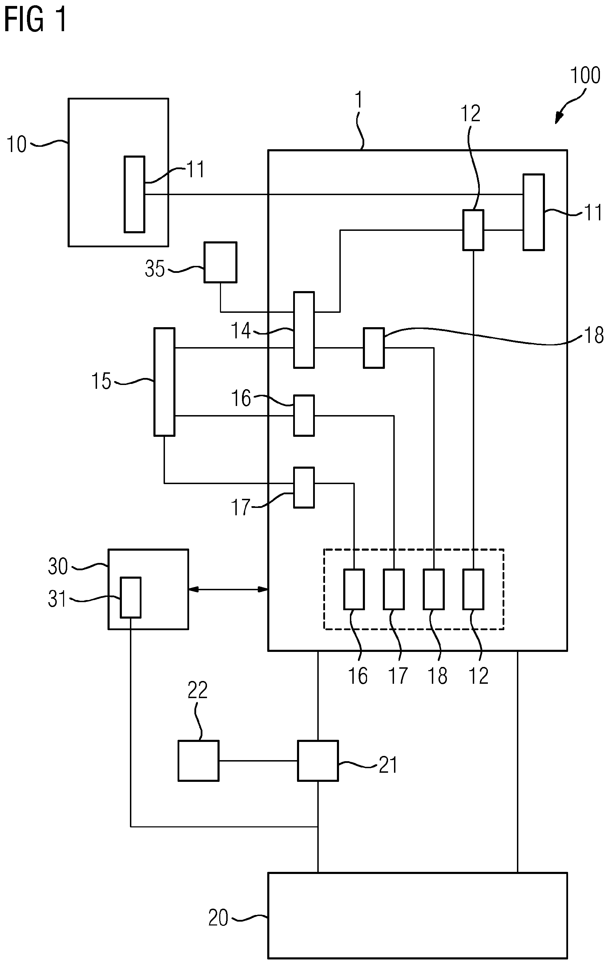

In figure 1 a block diagram illustrating a system 100 for analysing a medical imaging data set 11 according to a preferred embodiment of the present invention is shown. Such medical imaging data sets 11 are recorded and/or reconstructed by a medical imaging devices 10 such as a X-ray scanner, a computer tomography (CT) scanner, a magnetic resonance tomography (MRT) scanner or a ultrasound (US) scanner. Thus, it is possible to visualize inner parts of a patient for providing information that supports the clinical decision making process, i. e. a further treatment of the patient. For providing further relevant or needed information the medical imaging data set 10 is analysed by a specialist, such as a radiologist.

For optimizing the whole analyse procedure, in particular the workflow of analysing a plurality of medical imaging data sets, a control unit 1 for preselecting medical imaging data sets 11 is provided. The control unit 1 is configured for receiving the medical imaging data sets 11. In particular, the control unit 1 is part of a network, such as a cloud, or the control unit 2 is incorporated into a workstation, for example in a workstation of the medical imaging device, or into the medical imaging device 10. In the case of a control unit 2 being part of the network, the control unit 2 can receive medical imaging data sets 11 from different local medical imaging devices 10, for example located at different hospitals or different location within a hospital. In particular, the medical imaging device 10 and the control unit 2 are configured such that the medical imagine data set 11 is transferred to the control unit 1.

In particular, it is provided that a probability value 12 for a negative finding is assigned to the medical imaging data set 11, wherein the probability value 12 is based on the medical imaging data set 11. Preferably, for assigning a probability value 12 to the medical image data set 11 the control unit 1 comprises a trained artificial network 30 or is in communication with the artificial network 30, in particular an artificial network 30 trained by a machine learning mechanism such as a deep learning mechanism. As a consequence the control unit 1, in particular the trained artificial network 30, establishes a link between the medical imaging data set 11 and the probability value 12 for a negative finding, for example by identifying correlations between specific parameters of the medical imaging data set 11 and the probability for identifying no abnormality by using the specific medical imaging device. In other words: By using the control unit 1, in particular the artificial network 30 and a training data base 31 of the artificial network, a preselection of the medical imaging data sets 11 is possible, wherein the medical imaging data sets 11 are ranked by their respective probability value 14 assigned or related to each medical imaging data set 11. Thereby a training data base 31 is included to the artificial network 30 and/or the artificial network 30 is in communication with the training data base 30.

In addition to the medical imaging data set 11 an information data set 14 is transferred to the control unit 1. For example, the information data set 14 is added by entering information via an input device 15 and transferring the information data set 14 to the control unit 1. It is also thinkable that the information data set 14 is realized by extracting information from a patient related data base 35. For example, the control unit 1 extracts the information, in particular depending on the current medical imaging data set 11, from the patient related data base 35 automatically. The information data set 14 comprises patient information such as an age of the patient, a sex of the patient, a medical record of the patient and/or a lab information. In particular, it is provided that the information data set 14 is compiled automatically by accessing data from a RIS (radiological information system), a PACS (picture and communication system), a EMR (electronic medical record), a HIS (hospital information system), a LIS (laboratory information system) or comparable systems.

Further, the control unit 1 is configured for providing a probability value 12 based on the information data set 14 and/or the medical imaging data set 11. Thereby, the probability value 12 is individualized for each medical imaging data set 11 by assigning the probability value 12 to the medical imaging data set, in particular supported by the information data set 14 or by taking into account the information data set 14. For example, the probability value 12 is based on more than one, preferably more than three or even more than five parameter of the information data set 14 in addition to results of the pre-analysis of the medical imaging data set 11 by the control unit 1. It is also thinkable that the information data set 14 comprises a parameter specifying a disease or naming the disease. Another potential content of the information data set 14 might be an abnormality detected in the past.

Subsequently, the probability value 14 is compared to a threshold value 16. Based on this comparison, the control unit 1 automatically unit determines, whether the medical imaging data set 11 is transferred either
- to an output device 21 for analysing the medical imaging data set 11 or
- to a device for storing 20 the medical imaging data set 11
based on the probability value 12. In particular, the medical imaging data set 11 is transferred to the output device 21 for analysing the medical imaging data set 11, when the probability value 12 is smaller than the threshold value 16. The output device 21 might be a display or a printer for visual-ising the medical imaging data set 11. In particular, the output device 21 present the medical imaging data set to a specialist. In other words: the control unit 1 decides, whether the medical imaging data set 11 is presented to a specialist, for example a radiologist, for further analysis. As a result, the analyses of those medical imaging data sets 11 having a high probability for outputting a non-relevant result are skipped.

It is provided that the medical imaging data set 11 is directly transferred to a device 20 for storing the medical imaging data set 11, preferably device 20 for storing of a decision making support system, without further analysis, when the probability value 12 is greater than the threshold value 16, and is preferably further labelled with a standard phrase indicating that no further analysing of the medical imaging data 11 has been performed.

As a consequence, the total number of procedures over several analyses of different medical imaging data sets 11 can be reduced and therefore the system and/or the specialist for analysing the medical imaging data sets 11 is relieved.

Besides, a further probability value 18 is provided based on the information data set 14, in particular before recording the medical imaging data set 11, and wherein the medical imaging data set 11 is only automatically provided either
- to the output device 22 for analysing the medical imaging data set 11 or
- to the device for storing 20 the medical imaging data set 11
based on the probability value 12, when the difference between the threshold value 14 and the further probability 18 value is smaller than a further threshold 17. As a consequence, those medical imaging data sets 11 having a very low further probability are directly transferred to the output device 20 and preselecting by the control unit 1 is skipped. A very low further probability might be caused by a previous disease in the past. Thus, it is possible to make a preliminary selection for those medical imaging data sets 11 that definitively needs a detailed analysis or have such a high probability that there is no need for calculating the probability value 12 by the control unit 1. As a result, a transfer of those medical imaging data sets and information data sets 14, whose output of the control unit 1 is predictable, can be avoided, advantageously.

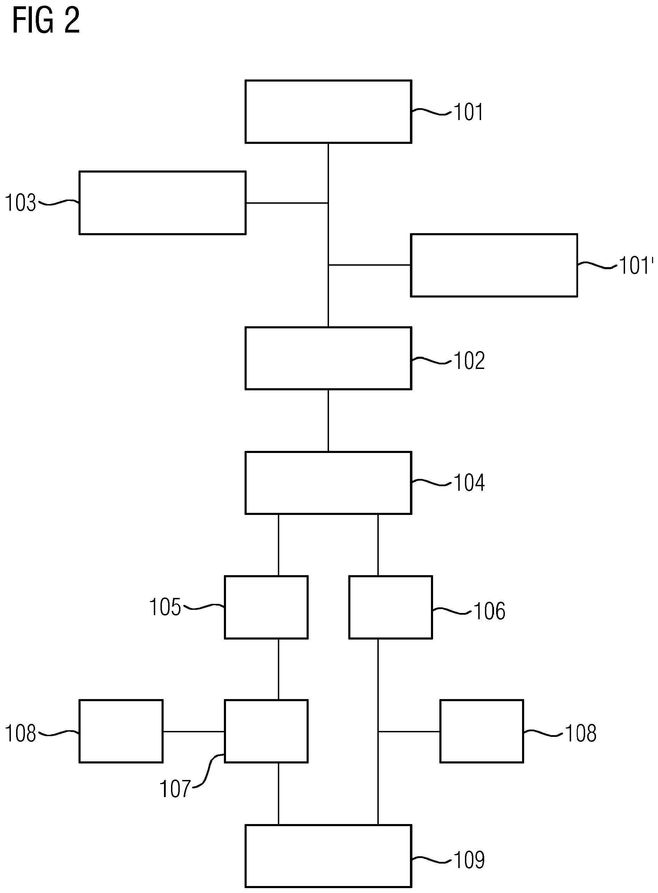

In figure 2 a flow diagram illustrating a method for analysing a medical imaging data set 11 according to a preferred embodiment of the present invention is illustrated. Thereby the method comprises:
- providing 101 a medical imaging data set 11,
- providing 101' the information data set 14,
- assigning 102 a probability value 12 to the medical imaging data set 11, in particular assigning 102 a probability value 12 based on the medical imaging data set 11 and/or the information data set 14 or assigning 102' a further probability value 18 to the information data set 14,
- configuring 103 the control unit 2, in particular by determining the threshold value 14 and the further threshold value 17,
- comparing 104 the probability value 12 to the threshold value 16 or comparing the difference of the further probability value 18 and the threshold value 16 to the further threshold value 17
- transferring 105 the medical imaging data set 11 to the output device 21 or transferring 106 the medical imaging data set 11 to the device 20 for storing the medical imaging data set 11 based on the probability value 14
- analysing 107 the medical imaging data set 11, in particular at the output device 21,
- training 108 the artificial network 30, in particular by the analysed medical imaging data sets 11,
- saving 109 the analysed medical imaging data set 11 to the device 20 for storing the medical imaging data sets.

## Claims

1. Method for analysing a medical imaging data set (11) comprising:
- providing the medical imaging data set (11);
- providing an information data set (14) compiling personal information of a patient;
- assigning a probability value (12) for a negative finding to the medical imaging data set (11), wherein the probability value (12) is based on the medical imaging data set (11) and the information data set (14) and represents a probability for successively identifying no abnormality by analysing the medical imaging data set (11) in detail;
- comparing the probability value (12) to a threshold value (16) ;
- providing a further probability value (18) based on the information data set (14); and
- providing the medical imaging data set (11) automatically either
- to an output device (21) for analysing the medical imaging data set (11) by an operator when the probability value (12) is smaller than the threshold value (16) or
- to a device (20) for storing the medical imaging data set (11) and/or for creating a report data set, thereby omitting the analysing step
based on the probability value (12) only when the difference between the threshold value (16) and the further probability value (18) is smaller than a further threshold (17).

2. Method according to claim 1, wherein the probability value (12) is provided by a trained artificial network (30), wherein preferably the artificial network (30) is trained by a machine leaning mechanism, in particular a deep learning mechanism, and/or by using a Siamese algorithm.

3. Method according to claim 2, wherein a result data set (40) comprising the medical imaging data set, the information data set and preferably the result of the detailed analysis is provided after analysing the medical imaging data set (11) and the result data set (40) is used for training the artificial network (30).

4. Method according to claim 3, wherein analysing is supported by an analysing device (22) for highlighting an abnormality.

5. Method according to claim 3 or 4, wherein the result data set (40) and/or the medical imaging data set (11) is transferred to a data base (22) of a clinical decision support system.

6. Method according to one of the preceding claims, wherein the medical imaging data set is recorded by a medical imaging device (10).

7. Method according to one of the preceding claims, wherein the information data set (14) and/or the threshold value (16) are entered via an input device (15).

8. Method according to one of the claims 1 to 6, wherein the information data set (14) and/or the threshold value (16) are set automatically, in particular by using the trained artificial network (30).

9. System (100) for analysing a medical imaging data set (11), wherein the system (100) is configured for:
- recording the medical imaging data set (11) by a medical imaging device (10);
- providing an information data set (14) compiling personal information of a patient;
- providing a probability value (12) for a negative finding wherein the probability value (12) is based on the medical imaging data set (11) and the information data set (14) and represents a probability for successively identifying no abnormality by analysing the medical imaging data set in detail; and
- comparing the probability value (12) to a threshold value (16) ;
- providing a further probability value (18) based on the information data set (14);
- providing the medical imaging data set (11) automatically either
- to an output device (21) for analysing the medical imaging data set by an operator when the probability value (12) is smaller than the threshold value (16) or
- to a device (20) for storing the medical imaging data set (11) and/or for creating a report data set, thereby omitting the analysing step,
based on the probability value (12) only when the difference between the threshold value (16) and the further probability value (18) is smaller than a further threshold (17)).

10. Computer program product comprising instructions which, when the program is executed by a computer, cause the computer to carry out the steps of the method according to any of claims 1 to 8.

11. A computer-readable medium on which are stored program elements that can be read and executed by a computer unit in order to perform steps of the method according to any of the claims 1 to 8 when the program elements are executed by the computer unit.

## Patentansprüche

1. Verfahren zum Analysieren eines medizinischen Bildgebungsdatensatzes (11) umfassend:
- Bereitstellen des medizinischen Bildgebungsdatensatzes (11);
- Bereitstellen eines Informationsdatensatzes (14), in dem persönliche Informationen eines Patienten zusammengestellt sind;
- Zuweisen eines Wahrscheinlichkeitswertes (12) für ein negatives Ergebnis zum medizinischen Bildgebungsdatensatz (11), wobei der Wahrscheinlichkeitswert (12) auf dem medizinischen Bildgebungsdatensatz (11) und dem Informationsdatensatz (14) basiert und eine Wahrscheinlichkeit für das erfolgreiche Identifizieren keiner Anomalie durch detailliertes Analysieren des medizinischen Bildgebungsdatensatzes (11) darstellt;
- Vergleichen des Wahrscheinlichkeitswertes (12) mit einem Schwellwert (16);
- Bereitstellen eines weiteren Wahrscheinlichkeitswertes (18) basierend auf dem Informationsdatensatz (14); und
- automatisches Bereitstellen des medizinischen Bildgebungsdatensatzes (11), entweder
- an eine Ausgabevorrichtung (21) zum Analysieren des medizinischen Bildgebungsdatensatzes (11) durch einen Bediener, wenn der Wahrscheinlichkeitswert (12) kleiner als der Schwellwert (16) ist, oder
- an eine Vorrichtung (20) zum Speichern des medizinischen Bildgebungsdatensatzes (11) und/oder zum Erstellen eines Berichtdatensatzes, wodurch der Analyseschritt basierend auf dem Wahrscheinlichkeitswert (12) nur ausgelassen wird, wenn die Differenz zwischen dem Schwellwert (16) und dem weiteren Wahrscheinlichkeitswert (18) kleiner als ein weiterer Schwellwert (17) ist.

2. Verfahren gemäß Anspruch 1, wobei der Wahrscheinlichkeitswert (12) von einem trainierten künstlichen Netzwerk (30) bereitgestellt wird, wobei das künstliche Netzwerk (30) vorzugsweise von einem Maschinenlernmechanismus trainiert wird, insbesondere einem Mechanismus für tiefes Lernen (Deep Learning), und/oder durch Verwenden eines siamesischen Algorithmus.

3. Verfahren gemäß Anspruch 2, wobei ein Ergebnisdatensatz (40), der den medizinischen Bildgebungsdatensatz, den Informationsdatensatz und vorzugsweise das Ergebnis der detaillierten Analyse umfasst, nach dem Analysieren des medizinischen Bildgebungsdatensatzes (11) bereitgestellt wird und der Ergebnisdatensatz (40) zum Trainieren des künstlichen Netzwerks (30) verwendet wird.

4. Verfahren gemäß Anspruch 3, wobei das Analysieren durch eine Analysevorrichtung (22) zum Hervorheben einer Anomalie unterstützt wird.

5. Verfahren gemäß Anspruch 3 oder 4, wobei der Ergebnisdatensatz (40) und/oder der medizinische Bildgebungsdatensatz (11) an eine Datenbank (22) eines klinischen Entscheidungsunterstützungssystems übertragen werden.

6. Verfahren gemäß einem der vorstehenden Ansprüche, wobei der medizinische Bildgebungsdatensatz von einer medizinischen Bildgebungsvorrichtung (10) aufgezeichnet wird.

7. Verfahren gemäß einem der vorstehenden Ansprüche, wobei der Informationsdatensatz (14) und/oder der Schwellwert (16) über eine Eingabevorrichtung (15) eingegeben werden.

8. Verfahren gemäß einem der vorstehenden Ansprüche 1 bis 6, wobei der Informationsdatensatz (14) und/oder der Schwellwert (16) automatisch eingestellt werden, insbesondere durch Verwenden des trainierten künstlichen Netzwerks (30).

9. System (100) zum Analysieren eines medizinischen Bildgebungsdatensatzes (11), wobei das System (100) ausgelegt ist zum:
- Aufzeichnen des medizinischen Bildgebungsdatensatzes (11) durch eine medizinische Bildgebungsvorrichtung (10);
- Bereitstellen eines Informationsdatensatzes (14), in dem persönliche Informationen eines Patienten zusammengestellt sind;
- Bereitstellen eines Wahrscheinlichkeitswertes (12) für ein negatives Ergebnis, wobei der Wahrscheinlichkeitswert (12) auf dem medizinischen Bildgebungsdatensatz (11) und dem Informationsdatensatz (14) basiert und eine Wahrscheinlichkeit für das erfolgreiche Identifizieren keiner Anomalie durch detailliertes Analysieren des medizinischen Bildgebungsdatensatzes darstellt; und
- Vergleichen des Wahrscheinlichkeitswerts (12) mit einem Schwellwert (16);
- Bereitstellen eines weiteren Wahrscheinlichkeitswertes (18) basierend auf dem Informationsdatensatz (14);
- automatisches Bereitstellen des medizinischen Bildgebungsdatensatzes (11), entweder
- an eine Ausgabevorrichtung (21) zum Analysieren des medizinischen Bildgebungsdatensatzes durch einen Bediener, wenn der Wahrscheinlichkeitswert (12) kleiner als der Schwellwert (16) ist, oder
- an eine Vorrichtung (20) zum Speichern des medizinischen Bildgebungsdatensatzes (11) und/oder zum Erstellen eines Berichtdatensatzes, wodurch der Analyseschritt basierend auf dem Wahrscheinlichkeitswert (12) nur ausgelassen wird, wenn die Differenz zwischen dem Schwellwert (16) und dem weiteren Wahrscheinlichkeitswert (18) kleiner als ein weiterer Schwellwert (17) ist.

10. Computerprogrammprodukt, das Anweisungen umfasst, die, wenn das Programm von einem Computer ausgeführt wird, den Computer veranlassen, die Schritte des Verfahrens gemäß einem der Ansprüche 1 bis 8 auszuführen.

11. Computerlesbares Medium, auf dem Programmelemente gespeichert sind, die von einer Computereinheit gelesen und ausgeführt werden können, um Schritte des Verfahrens gemäß einem der Ansprüche 1 bis 8 durchzuführen, wenn die Programmelemente von der Computereinheit ausgeführt werden.

## Revendications

1. Procédé d'analyse d'un ensemble (11) de données d'imagerie médicale comprenant :
- on se procure l'ensemble (11) de données d'imagerie médicale ;
- on se procure un ensemble (14) de données d'information compilant de l'information personnelle sur un patient ;
- on affecte une valeur (12) de probabilité d'une constatation négative à l'ensemble (11) de données d'imagerie médicale, dans lequel la valeur (12) de probabilité repose sur l'ensemble (11) de données d'imagerie médicale et sur l'ensemble (14) de données d'information et représente une probabilité pour identifier avec succès qu'il n'y a pas d'anomalie en analysant l'ensemble (11) de données d'imagerie médicale en détail ;
- on compare la valeur (12) de probabilité à une valeur (16) de seuil ;
- on se procure une autre valeur (18) de probabilité reposant sur l'ensemble (14) de données d'information ; et
- on fournit l'ensemble (11) de données d'imagerie médicale automatiquement soit
- à un dispositif (21) de sortie pour analyser l'ensemble (11) de données d'imagerie médicale par un opérateur, lorsque la valeur (12) de probabilité est plus petite que la valeur (16) de seuil, soit
- à un dispositif (20) de mise en mémoire de l'ensemble (11) de données d'imagerie médicale et/ou de création d'un ensemble de données de compte rendu, en omettant ainsi le stade d'analyse,
reposant sur la valeur (12) de probabilité, seulement lorsque la différence entre la valeur (16) de seuil et l'autre valeur (18) de probabilité est plus petite qu'un autre seuil (17).

2. Procédé suivant la revendication 1, dans lequel la valeur (12) de probabilité est fournie par un réseau (30) artificiel ayant subi un apprentissage, dans lequel, de préférence, le réseau (30) artificiel subit un apprentissage par un mécanisme d'apprentissage automatique, en particulier par un mécanisme d'apprentissage profond et/ou en utilisant un algorithme de Siamese.

3. Procédé suivant la revendication 2, dans lequel un ensemble (40) de données de résultat comprenant l'ensemble de données d'imagerie médicale, l'ensemble de données d'information et, de préférence, le résultat de l'analyse détaillée, est obtenu après avoir analysé l'ensemble (11) de données d'imagerie médicale et l'ensemble (40) de données de résultat est utilisé pour faire l'apprentissage du réseau (30) artificiel.

4. Procédé suivant la revendication 3, dans lequel l'analyse est assistée par un dispositif (22) d'analyse pour mettre en lumière une anomalie.

5. Procédé suivant la revendication 3 ou 4, dans lequel l'ensemble (40) de données de résultat et/ou l'ensemble (11) de données d'imagerie médicale est transféré à une base (22) de données d'un système d'aide à la décision clinique.

6. Procédé suivant l'une des revendications précédentes, dans lequel on enregistre l'ensemble de données d'imagerie médicale par un dispositif (10) d'imagerie médicale.

7. Procédé suivant l'une des revendications précédentes, dans lequel on entre l'ensemble (14) de données d'information et/ou la valeur (16) de seuil, par l'intermédiaire d'un dispositif (15) d'entrée.

8. Procédé suivant l'une des revendications 1 à 6, dans lequel on règle automatiquement l'ensemble (14) de données d'information et/ou la valeur (16) de seuil, en particulier en utilisant le réseau (30) artificiel ayant subi un apprentissage.

9. Système (100) d'analyse d'un ensemble (11) de données d'imagerie médicale, dans lequel le système (100) est configuré pour :
- enregistrer l'ensemble (11) de données d'imagerie médicale par un dispositif (10) d'imagerie médicale ;
- fournir un ensemble (14) de données d'information compilant de l'information personnelle sur un patient ;
- fournir une valeur (12) de probabilité d'une constatation négative, dans lequel la valeur (12) de probabilité repose sur l'ensemble (11) de données d'imagerie médicale et sur l'ensemble (14) de données d'information et représente une probabilité d'identifier avec succès qu'il n'y a pas d'anomalie en analysant l'ensemble de données d'imagerie médicale en détail ; et
- comparer la valeur (12) de probabilité à une valeur (16) de seuil ;
- fournir une autre valeur (18) de probabilité reposant sur l'ensemble (14) de données d'information ;
- fournir l'ensemble (11) de données d'imagerie médicale automatiquement soit
- à un dispositif (21) de sortie pour analyser l'ensemble de données d'imagerie médicale par un opérateur, lorsque la valeur (12) de probabilité est plus petite que la valeur (16) de seuil, soit
- à un dispositif (20) de mise en mémoire de l'ensemble (11) de données d'imagerie médicale et/ou de création d'un ensemble de données de compte rendu, en omettant ainsi le stade d'analyse,
reposant sur la valeur (12) de probabilité, seulement lorsque la différence entre la valeur (16) de seuil et l'autre valeur (18) de probabilité est plus petite qu'un autre seuil (17).

10. Produit de programme d'ordinateur comprenant des instructions qui, lorsque le programme est exécuté par un ordinateur, font que l'ordinateur effectue les stades du procédé suivant l'une quelconque des revendications 1 à 8.

11. Support, déchiffrable par ordinateur, sur lequel sont mis en mémoire des éléments de programme, qui peuvent être déchiffrés et exécutés par une unité informatique, afin d'effectuer des stades du procédé suivant l'une quelconque des revendications 1 à 8, lorsque les éléments du programme sont exécutés par l'unité informatique.
